(19) Europäisches Patentamt
European Patent Office
Office européen des brevets

(11) EP 3 020 732 B1

(12) **EUROPEAN PATENT SPECIFICATION**

(45) Date of publication and mention of the grant of the patent:
**18.03.2020 Bulletin 2020/12**

(21) Application number: **14822721.8**

(22) Date of filing: **14.07.2014**

(51) Int Cl.:
***C07K 19/00*** *(2006.01)* ***C07K 16/46*** *(2006.01)*
***C07K 14/72*** *(2006.01)*

(86) International application number:
**PCT/KR2014/006328**

(87) International publication number:
**WO 2015/005747 (15.01.2015 Gazette 2015/02)**

# (54) AN IMMUNOGLOBULIN FC CONJUGATE WHICH MAINTAINS BINDING AFFINITY OF IMMUNOGLOBULIN FC FRAGMENT TO FCRN

EIN IMMUNOGLOBULIN-FC-KONJUGAT, WELCHES DIE BINDUNGSAFFINITÄT VON IMMUNOGLOBULIN-FC-FRAGMENT ZU FCRN BEIBEHÄLT

CONJUGUÉ DE RÉGION FC D'IMMUNOGLOBULINE MAINTENANT L'AFFINITÉ DE LIAISON DU FRAGMENT FC DE L'IMMUNOGLOBULINE À FCRN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.07.2013 KR 20130082509**

(43) Date of publication of application:
**18.05.2016 Bulletin 2016/20**

(73) Proprietor: **Hanmi Pharm. Co., Ltd.**
**Hwaseong-si, Gyeonggi-do 445-858 (KR)**

(72) Inventors:
- **HWANG, Sang Youn**
  **Hwaseong-si**
  **Gyeonggi-do 445-769 (KR)**
- **LEE, Jong Soo**
  **Seongnam-si**
  **Gyeonggi-do 463-725 (KR)**
- **HONG, Sung Hee**
  **Suwon-si**
  **Gyeonggi-do 443-769 (KR)**
- **CHOI, In Young**
  **Yongin-si**
  **Gyeonggi-do 448-160 (KR)**
- **JUNG, Sung Youb**
  **Hwaseong-si**
  **Gyeonggi-do 18469 (KR)**
- **KWON, Se Chang**
  **Seoul 135-506 (KR)**

(74) Representative: **Mewburn Ellis LLP**
**City Tower**
**40 Basinghall Street**
**London EC2V 5DE (GB)**

(56) References cited:
**WO-A1-2005/047336** **WO-A1-2006/107124**
**WO-A1-2008/136611** **WO-A2-2010/011096**
**WO-A2-2011/122921** **WO-A2-2012/165915**
**KR-A- 20060 106 486** **KR-A- 20090 045 953**
**KR-A- 20120 014 936** **KR-A- 20120 041 139**
**KR-A- 20120 138 241**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 020 732 B1

Printed by Jouve, 75001 PARIS (FR)

## Description

[Technical Field]

The present disclosure relates to a physiologically active polypeptide-immunoglobulin Fc fragment conjugate comprising a physiologically active polypeptide linked via a non-peptidyl linker to an immunoglobulin Fc fragment with an FcRn-binding region capable of maintaining the intrinsic binding affinity of the immunoglobulin Fc fragment, a method for preparing the conjugate, a method of maintaining the intrinsic binding affinity of the conjugate for FcRn, and a composition comprising the conjugate capable of maintaining the intrinsic binding affinity of the immunoglobulin Fc fragment for FcRn.

[Background Art]

Various studies have been conducted on protein conjugates or complexes that comprise a carrier, such as a polyethylene glycol polymer, albumin, fatty acid or antibody Fc (constant region), linked to a protein in order to increase the serum half-life of the protein. Studies known to date about such protein conjugates or complexes mostly aim to increase the serum half-life of a drug to shorten the interval of drug administration to thereby improve patient convenience. However, many conventional technologies have problems such as a decrease in the activity of a therapeutic protein due to, for example, a spatial hindrance caused by a non-specific binding between a therapeutic protein and a carrier protein. In addition, in the case of fatty acid conjugates that reversibly bind to serum albumin to increase their serum half-life, there is a limit to significantly increase the serum half-life of a protein drug, because renal clearance, which accounts for the greatest loss of a protein drug, cannot be avoided due to the reversible binding between the protein and the fatty acid.

Moreover, efforts have been made to use immunoglobulin fragments to increase the half-life of physiologically active substances including proteins. The CH2-CH3 region of immunoglobulin Fc includes an FcRn (protection receptor)-binding site that prolongs the half-life of an antibody. FcRn is an MHC class I-related protein expressed in vascular endothelial cells and binds to IgG and albumin. Characteristically, IgG and FcRn strongly bind to each other at a weak acidic pH and dissociate from each other at a neutral pH. Thus, when IgG enters vascular endothelial cells from blood vessels by pinocytosis or endocytosis and enters lysosomes (pH 6.0), it is protected by FcRn without being degraded. When IgG is fused with the cell membrane by recycling, it dissociates from FcRn at pH 7.4 and is released into blood vessels. Due to this procedure, the *in vivo* half-lives of IgG1, IgG2 and IgG4, which include the FcRn-binding site, are 3 weeks on average, being longer than those of other proteins.

Thus, when an immunoglobulin Fc fragment is linked to a physiologically active substance, the half-life of the physiologically active substance can be increased by FcRn-mediated recycling. In this regard, there is a need for the development of a method capable of maintaining the binding affinity of an immunoglobulin Fc fragment for FcRn without reducing the activity of a physiologically active substance when the physiologically active substance and the immunoglobulin Fc fragment are linked together.

[Disclosure]

[Technical Problem]

The present authors have made extensive efforts to develop a conjugate that can maintain the intrinsic binding affinity of an immunoglobulin Fc fragment itself for FcRn without reducing the activity of a physiologically active polypeptide and can easily dissociate from FcRn at a neutral pH. As a result, the present authors have found that a conjugate comprising a physiologically active polypeptide linked via a non-peptidyl linker to an immunoglobulin Fc fragment having an FcRn-binding region can maintain the intrinsic binding affinity of the immunoglobulin Fc fragment for FcRn and, at the same time, can easily dissociate from FcRn at a neutral pH of 7.4, thereby completing the present disclosure.

[Technical Solution]

It is an objective of the present disclosure to provide a physiologically active polypeptide-immunoglobulin Fc fragment conjugate that comprises a physiologically active polypeptide linked via a non-peptidyl linker to an immunoglobulin Fc fragment comprising an FcRn-binding region, wherein the binding ratio of the amount of the conjugate bound to FcRn at pH 6.0 and the amount of the conjugate bound to FcRn at pH 7.4 is within the range of ± 6% of a ratio determined by measuring the amounts of binding of the immunoglobulin Fc fragment to FcRn under the same conditions as those used for the conjugate.

Another objective of the present disclosure is to provide a physiologically active polypeptide-immunoglobulin

Fc fragment conjugate that comprises a physiologically active polypeptide linked via a non-peptidyl linker to an immunoglobulin Fc fragment comprising an FcRn-binding region, wherein a binding ratio determined by substituting the amount of the conjugate bound to FcRn at pH 6.0 and the amount of the conjugate bound to FcRn at pH 7.4 into the following equation 1 is within the range of ± 6% of a binding ratio determined by measuring the amounts the immunoglobulin Fc fragment bound to FcRn under the same conditions as those used for the conjugate:

Equation 1

$$\text{Binding ratio (\%)} = (\text{amount bound at pH 7.4/ amount bound at pH 6.0}) \times 100.$$

Still another objective of the present disclosure is to provide a method for preparing a physiologically active polypeptide-immunoglobulin Fc fragment conjugate that maintains the intrinsic binding affinity of an immunoglobulin Fc fragment for FcRn, the method comprising: (a) linking a physiologically active polypeptide via a non-peptidyl linker to an immunoglobulin Fc fragment having an FcRn-binding region to prepare a mixture of physiologically active polypeptide-immunoglobulin Fc fragment conjugates; and (b) separating from the mixture a physiologically active polypeptide-immunoglobulin Fc fragment conjugate that shows a binding ratio within the range of ± 6% of the binding ratio of the immunoglobulin Fc fragment, as determined by substituting the amount bound to FcRn at pH 6.0 and the amount bound to FcRn at pH 7.4 into equation 1.

Still another objective of the present disclosure is to provide a method of maintaining the intrinsic binding affinity of a physiologically active polypeptide-immunoglobulin Fc fragment conjugate for FcRn, the method comprising linking a physiologically active polypeptide via a non-peptidyl linker to an immunoglobulin Fc fragment comprising an FcRn-binding region.

Still another objective of the present disclosure is to provide a composition comprising the physiologically active polypeptide-immunoglobulin Fc fragment conjugate that maintains the intrinsic binding affinity of the immunoglobulin Fc fragment for FcRn.

[Advantageous Effects]

As described above, the conjugate of the present disclosure maintains the intrinsic binding affinity of the immunoglobulin Fc fragment for FcRn and, at the same time, easily dissociates from FcRn at a neutral pH such as a pH of 7.4. Thus, it can be advantageously used to increase the serum half-life of a physiologically active polypeptide.

[Description of Drawings]

FIG. 1 is a schematic view showing the process of recycling a protein bound to FcRn.
FIG. 2 shows sensograms of the binding of immunoglobulin-protein conjugates to FcRn at an acidic pH.
FIG. 3 shows sensograms of the binding of immunoglobulin-protein conjugates to FcRn at a neutral pH.
FIG. 4 shows the results of a test for comparison of *in vivo* pharmacokinetics of GLP-1R agonist-immunoglobulin fragment conjugate

[Best Mode]

To achieve the above objects, in one aspect, the present disclosure provides a physiologically active polypeptide-immunoglobulin Fc fragment conjugate that comprises a physiologically active polypeptide linked via a non-peptidyl linker to an immunoglobulin Fc fragment comprising an FcRn-binding region, wherein the binding ratio of the amount of the conjugate bound to FcRn at pH 6.0 and the amount of the conjugate bound to FcRn at pH 7.4 is within the range of ± 6% of a ratio determined by measuring the amounts of the immunoglobulin Fc fragment bound to FcRn under the same conditions as those used for the conjugate.

In one embodiment, the conjugate according to the present disclosure shows a binding ratio within the range of ± 6% of the binding ratio of the immunoglobulin Fc fragment, as determined using the following equation:

Equation 1

$$\text{Binding ratio (\%)} = (\text{amount bound at pH 7.4/ amount bound at pH 6.0}) \times 100.$$

In another embodiment, the conjugate according to the present disclosure may be obtained by reacting the physiologically active polypeptide having the non-peptidyl linker linked thereto with the immunoglobulin Fc fragment at a pH of 4.0-9.0, thereby linking the physiologically active polypeptide via the non-peptidyl linker to a portion excluding the FcRn-binding region of the immunoglobulin Fc fragment.

In still another embodiment, the non-peptidyl linker that is included in the conjugate according to the present disclosure may be selected from the group consisting of polyethylene glycol, polypropylene glycol, an ethylene glycol-propylene glycol copolymer, polyoxyethylated polyol, polyvinyl alcohol, polysaccharide, dextran, polyvinyl ethyl ether, a biodegradable polymer, a lipid polymer, chitin, hyaluronic acid, and combinations thereof

In still another embodiment, the non-peptidyl linker that is included in the conjugate according to the present disclosure may be a polyethylene glycol polymer represented by the following formula 1:

Formula 1

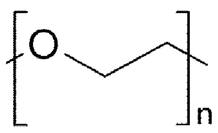

wherein n ranges from 10 to 2400.

In still another embodiment, the non-peptidyl linker that is included in the conjugate according to the present disclosure may have a reactive group selected from the group consisting of an aldehyde group, a propionaldehyde group, a butyraldehyde group, a maleimide group, and succinimide derivatives.

In still another embodiment, the physiologically active polypeptide that is included in the conjugate according to the present disclosure may be selected from the group consisting of glucagon-like peptide-1 (GLP-1), granulocyte colony stimulating factor (G-CSF), human growth hormone (hGH), erythropoietin (EPO), glucagon, oxyntomodulin, insulin, growth hormone releasing hormone, growth hormone releasing peptide, interferons, interferon receptors, G-protein-coupled receptor, interleukins, interleukin receptors, enzymes, interleukin binding proteins, cytokine binding proteins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin, $\alpha$-lactalbumin, apolipoprotein-E, highly glycosylated erythropoietin, angiopoietins, hemoglobin, thrombin, thrombin receptor activating peptide, thrombomodulin, blood factors VII, VIIa, VIII, IX and XIII, plasminogen activating factor, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone stimulating protein, calcitonin, atriopeptin, cartilage inducing factor, elcatonin, connective tissue activating factor, tissue factor pathway inhibitor, follicle stimulating hormone, luteinizing hormone, luteinizing hormone releasing hormone, nerve growth factors, parathyroid hormone, relaxin, secretin, somatomedin, insulin-like growth factor, adrenocortical hormone, cholecystokinin, pancreatic polypeptide, gastrin releasing peptide, corticotropin releasing factor, thyroid stimulating hormone, autotaxin, lactoferrin, myostatin, cell surface antigens, virus derived vaccine antigens, monoclonal antibodies, polyclonal antibodies, and antibody fragments.

In still another embodiment, the immunoglobulin Fc fragment comprising the FcRn-binding region, which is included in the conjugate according to the present disclosure, may comprise a CH2 domain, a CH3 domain, or both.

In still another embodiment, the immunoglobulin Fc fragment that is included in the conjugate according to the present disclosure may be in a non-glycosylated form.

In still another embodiment, the immunoglobulin Fc fragment that is included in the conjugate according to the present disclosure may comprise a hinge region.

In still another embodiment, the immunoglobulin Fc fragment that is included in the conjugate according to the present disclosure may be selected from the group consisting of IgG, IgA, IgD, IgE, IgM, combinations thereof, and hybrids thereof.

In still another embodiment, the immunoglobulin Fc fragment that is included in the conjugate according to the present disclosure may be an IgG4 Fc fragment.

In another aspect, the present disclosure provides a method for preparing a physiologically active polypeptide-immunoglobulin Fc fragment conjugate that maintains the intrinsic binding affinity of an immunoglobulin Fc fragment for FcRn, the method comprising: (a) linking a physiologically active polypeptide via a non-peptidyl linker to an immunoglobulin Fc fragment comprising an FcRn-binding region to a physiologically active polypeptide via a non-peptidyl linker to prepare a mixture of physiologically active polypeptide-immunoglobulin Fc fragment conjugates; and (b) separating from the mixture a physiologically active polypeptide-immunoglobulin Fc fragment conjugate that shows a binding ratio within the range of $\pm$ 6% of the binding ratio of the immunoglobulin Fc fragment, as determined by substituting the amount of

binding to FcRn at pH 6.0 and the amount of binding to FcRn at pH 7.4 into equation 1.

In one embodiment, the non-peptidyl linker that is used in the preparation method according to the present disclosure may be a polyethylene glycol polymer represented by the following formula 1:

Formula 1

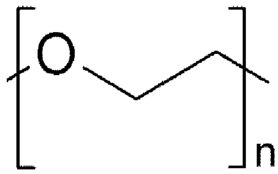

wherein n ranges from 10 to 2400.

In still another embodiment, the conjugate separated in step (b) of the preparation method according to the present disclosure may have a structure in which the non-peptidyl linker is bound to the N-terminus of the immunoglobulin Fc fragment.

In still another aspect, the present disclosure provides a method of maintaining the intrinsic binding affinity of a physiologically active polypeptide-immunoglobulin Fc fragment conjugate for FcRn, the method comprising linking a physiologically active polypeptide via a non-peptidyl linker to an immunoglobulin Fc fragment comprising an FcRn-binding region.

In one embodiment, the maintaining of the intrinsic binding affinity may be effected *in vivo.*

In still another aspect, the present disclosure provides a composition comprising the physiologically active polypeptide-immunoglobulin Fc fragment conjugate, which maintains the intrinsic binding affinity of the immunoglobulin Fc fragment for FcRn.

[Mode for Disclosure]

In one aspect, the present disclosure provides a physiologically active polypeptide-immunoglobulin Fc fragment conjugate that comprises a physiologically active polypeptide linked via a non-peptidyl linker to an immunoglobulin Fc fragment comprising an FcRn-binding region, wherein the ratio of the amount of the conjugate bound to FcRn at pH 6.0 and the amount of the conjugate bound to FcRn at pH 7.4 is within the range of ± 6% of a ratio determined by measuring the amounts of the immunoglobulin Fc fragment bound to FcRn under the same conditions as those used for the conjugate.

As used herein, the expression "amount of binding of the physiologically active polypeptide-immunoglobulin Fc fragment conjugate" refers to the ratio of the concentration of the conjugate protein bound to FcRn relative to the total concentration of the conjugate protein bound or unbound to FcRn at the pH of interest. In the present disclosure, because this amount of binding is used to calculate the ratio between the amounts of binding at two pHs, the ratio between the amounts of binding may be calculated from the absolute amount of binding measured at one pH and may also be determined by measuring other physical amounts, which are proportional to the amount of the bound conjugate and easy to measure. For example, the ratio between the amounts of binding can be determined by measuring surface plasmon resonance (SPR) signals and calculating the ratio between the signals at pH 6.0 and pH 7.4. In addition, other methods may also be used to determine the ratio between the amounts of binding.

Specifically, the present disclosure provides a physiologically active polypeptide-immunoglobulin Fc fragment conjugate that comprises a physiologically active polypeptide linked via a non-peptidyl linker to an immunoglobulin Fc fragment comprising an FcRn-binding region, wherein a binding ratio determined by substituting the amount of the conjugate bound to FcRn at pH 6.0 and the amount of the conjugate bound to FcRn at pH 7.4 into the following equation 1 is less than the range of ± 6% of a binding ratio determined by measuring the amounts of the immunoglobulin Fc fragment bound to FcRn under the same conditions as those used for the conjugate:

Equation 1

$$\text{Binding ratio (\%)} = (\text{amount bound at pH 7.4/ amount bound at pH 6.0}) \times 100$$

The binding ratio can be obtained as a percentage (%) value by dividing the amount of the conjugate (or immunoglobulin Fc fragment) binding to FcRn at pH 7.4 by the amount of the conjugate (or immunoglobulin Fc fragment) binding to FcRn at pH 6.0 and multiplying the divided value by 100.

The binding ratio can be calculated using the method proposed by Weirong Wang et al. (DRUG METABOLISM

AND DISPOSITION 39:1469-1477, 2011) for predicting the half-life of a monoclonal antibody, but is not specifically limited thereto. In the method described in the above literature, a monoclonal antibody having a high binding ratio showed a short *in vivo* half life.

The method for measuring the binding ratio may be performed, particularly using a surface plasmon resonance method, but is not specifically limited thereto.

For example, the method for measuring the binding ratio can be performed by immobilizing FcRn onto a biosensor chip (e.g., a Biacore CM5 biosensor chip) using an amine coupling kit or the like, injecting the FcRn-immobilized biosensor chip with an acidic buffer (e.g., pH 6.0 buffer) containing a material (e.g., the conjugate or the immunoglobulin Fc fragment) to be measured for the extent of binding to FcRn, and then injecting a neutral buffer (e.g., pH 7.4 buffer) into the biosensor chip. In this case, the binding ratio can be determined by measuring the resonance unit (RU) in an equilibrium state before completion of the injection of the sample in the pH 6.0 buffer into the chip to determine the amount bound at pH 6.0, measuring the resonance unit after injection of the pH 7.4 buffer to determine the amount bound at pH 7.4, and then dividing the amount bound at pH 7.4 by the amount bound at pH 6.0. When the binding ratio is to be expressed in percentage, the divided value can be multiplied by 100.

In the above-described method, the composition of the pH 6.0 buffer is not specifically limited, as long as it can induce a binding between the material to be measured for the extent of binding to FcRn and FcRn. For example, the pH 6.0 buffer may be a buffer containing a salt such as phosphate. The concentration of the salt in the buffer may be 50-200 mM, but is not limited thereto. Also, the buffer may be injected into the FcRn-immobilized biosensor chip at a temperature of about 25 °C, but the temperature of measurement may be changed within the temperature range in which the pH of the buffer does not change.

In addition, the pH 6.0 buffer may contain the material to be measured for the extent of binding to FcRn. The concentration of the material to be measured for the degree of binding to FcRn in the pH 6.0 buffer is not specifically limited, as long as the extent of binding to FcRn can be measured. For example, the concentration of the material to be measured for the degree of binding to FcRn in the pH 6.0 buffer may be between 100 nM and 12.5 nM.

In the above-described method, the composition of the pH 7.4 buffer is not specifically limited, as long as it can induce dissociation between the material to be measured for the extent of binding to FcRn and FcRn. For example, the pH 7.4 buffer may be a buffer containing phosphate. The concentration of a salt in the buffer may be 50-200 mM, but is not limited thereto. Also, the pH 7.4 buffer may be injected into the FcRn-immobilized biosensor chip at a temperature of 25~37 °C, but is not limited thereto. In a specific embodiment, the ratio between the amounts of binding is measured at a temperature of 25 °C.

Moreover, the amount of binding between FcRn and the material to be measured for the extend of binding to FcRn at pH 6.0 may be a resonance unit value measured at 2-60 seconds before the completion of injection of the pH 6.0 sample. Binding between FcRn and the material to be measured for the extent of binding to FcRn is preferably in an equilibrium state at the time point of measurement.

Also, the amount of binding between FcRn and the material to be measured for the extent of binding to FcRn at pH 7.4 may be a resonance unit value measured at 10-20 seconds after injection of the pH 7.4 buffer. The time point of measurement is preferably between the time point at which the RU value changes rapidly and before the RU value reaches 0.

In a comparison of the binding ratio between the immunoglobulin Fc fragment and the conjugate according to the present disclosure, the binding ratios are preferably values determined by the same experimental method under the same experimental conditions, but the compositions and temperatures of the buffers may vary depending on the kind of conjugate.

The surface plasmon resonance method as described above is one of methods for determining the binding ratio. In addition to the surface plasmon resonance method, any method may be used in the present disclosure, as long as it is a method such as an enzyme-immunosorbent assay (ELISA), which can measure the amount of the conjugate bound to FcRn. In addition, the unit of the amount of binding may vary depending on the method used.

When the binding ratio of a physiologically active polypeptide-immunoglobulin Fc fragment conjugate that comprises a physiologically active polypeptide linked via a non-peptidyl linker to an immunoglobulin Fc fragment having an FcRn-binding region is within the range of $\pm$ 6.0% of the binding ratio of the immunoglobulin Fc fragment itself, as measured by the above-described method, the physiologically active polypeptide-immunoglobulin Fc fragment conjugate has a long *in vivo* half-life.

In the present disclosure, it was found that, even when a physiologically active polypeptide was covalently linked via a non-peptidyl linker to an immunoglobulin Fc fragment having an FcRn-binding region to form a conjugate, the intrinsic binding affinity of the immunoglobulin Fc fragment for FcRn could be maintained, suggesting that intracellular recycling of the immunoglobulin Fc fragment can easily occur to increase the *in vivo* half-life. Particularly, when the non-peptidyl linker binds to the portion excluding the FcRn-binding region of the immunoglobulin Fc fragment, it does not reduce the binding affinity of the immunoglobulin Fc fragment for FcRn. When the non-peptidyl linker is polyethylene glycol ($-[O-CH_2-CH_2]n-$), n in $-[O-CH_2-CH_2]n-$ is 10 or greater, particularly 10-2400, more particularly 10-480, and even

more particularly 50-250, but is not limited thereto.

It was found that when n in $-[O-CH_2-CH_2]n-$ is particularly 10-2400, and more particularly 50-2400, the polyethylene glycol does not influence the physiologically activity and FcRn-binding activity of each of the physiologically active polypeptide and the immunoglobulin Fc fragment. The present disclosure is based on this characteristic.

As used herein, the term "physiologically active polypeptide-immunoglobulin Fc fragment conjugate" refers to a conjugate that comprises a physiologically active polypeptide covalently linked via a non-peptidyl linker to an immunoglobulin Fc fragment having an FcRn-binding region and shows a binding ratio within the range of ± 6.0% of the binding ratio of the immunoglobulin Fc fragment, as determined by substituting the amount of binding to FcRn at pH 6.0 and the amount of binding to FcRn at pH 7.4 into equation 1.

The non-peptidyl linker in the conjugate may be linked to amino acid residues away from the FcRn-binding region of the immunoglobulin Fc fragment, for example, a region corresponding to positions 252-257 and 307-311 of CH2 and positions 433-436 of CH2, numbered according to the Kabat numbering system. The non-peptidyl linker may preferably be linked to the N-terminus or C-terminus of the immunoglobulin Fc fragment, and more preferably linked to the N-terminus, but is not limited thereto.

When the non-peptidyl linker is linked to the N-tenninus or C-tenninus of the immunoglobulin Fc fragment, it does not substantially reduce the binding affinity of the immunoglobulin Fc fragment for FcRn, and thus the intrinsic binding affinity of the immunoglobulin Fc fragment of the conjugate for FcRn can be maintained.

As used herein, the term "non-peptidyl linker" refers to a biocompatible polymer composed of two or more repeating units linked to each other, in which the repeating units are linked to each other by any non-peptide covalent bond. This non-peptidyl linker may have two or three ends.

The non-peptidyl linker used in the present disclosure may be selected from the group consisting of biodegradable polymers such as polyethylene glycol, polypropylene glycol, a copolymer of ethylene glycol with propylene glycol, polyoxyethylated polyol, polyvinyl alcohol, polysaccharide, dextran, polyvinyl ethyl ether, biodegradable polymers such as polylactic acid (PLA) and polylactic-glycolic acid (PLGA), lipid polymers, chitins, hyaluronic acid, and combinations thereof, but is not limited thereto. Preferably, the non-peptidyl linker is polyethylene glycol, for example, a polyethylene glycol polymer represented by the following formula 1, but is not limited:

Formula 1

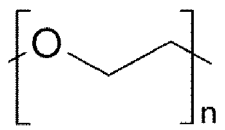

wherein n ranges from 10 to 2400, preferably from 10 to 480, and more preferably from 50 to 250, but is not limited thereto.

Meanwhile, other non-peptidyl linkers having a molecular weight corresponding to that of the polyethylene glycol of formula 1 also fall within the scope of the present disclosure.

In addition, their derivatives known in the art and non-peptidyl linker derivatives that can be easily prepared in the state of the art also fall within the scope of the present disclosure.

Polyethylene glycol that is used as the non-peptidyl linker in the present disclosure has an advantage in that it does not result in spatial hindrance between the physiologically active polypeptide and immunoglobulin Fc fragment bound to both ends thereof so that both the physiological activity of the physiologically active polypeptide and the binding affinity of the immunoglobulin Fc fragment for FcRn can be maintained.

A peptidyl linker that is used in a fusion protein prepared by a conventional in-frame fusion method has a disadvantage in that it is easily cleaved by protease *in vivo,* and thus the expected effect of increasing the serum half-life of the active drug by a carrier cannot be obtained. However, the conjugate comprising the non-peptidyl linker according to the present disclosure dramatically overcomes this disadvantage. The non-peptidyl linker may be a polymer that has resistance to protease to maintain the serum half-life of the peptide, similar to that of a carrier. Therefore, any non-peptidyl linker may be used in the present disclosure without any limitation, as long as it is a polymer having the above-described function, that is, a polymer having resistance to protease *in vivo.*

In addition, the non-peptidyl linker that is linked to the immunoglobulin Fc fragment in the present disclosure may be made not only of one kind of polymer, but also of a combination of different kinds of polymers.

The non-peptidyl linker that is used in the present disclosure has reactive groups capable of binding to the immunoglobulin Fc fragment and the physiologically active polypeptide.

The reactive groups at both ends of the non-peptidyl polymer are preferably selected from the group consisting of a reactive aldehyde group, a propionaldehyde group, a butyraldehyde group, a maleimide group, and succinimide derivative. Herein, the succinimide derivative may be succinimidyl propionate, hydroxy succinimidyl, succinimidyl carboxymethyl, or succinimidyl carbonate. In particular, when the non-peptidyl polymer has a reactive aldehyde group at both ends thereof, a physiologically active polypeptide and an immunoglobulin effectively bind to both ends of the non-peptidyl linker, respectively, while minimizing non-specific reactions. A final product generated by reductive alkylation via an aldehyde bond is much more stable than that linked via an amide bond. The aldehyde reactive group can bind selectively to the N-terminus at a low pH and can form a covalent bond with a lysine residue at a high pH, for example, at pH 9.0. Herein, the non-peptidyl linker may contain two or more aldehyde groups or have two or more alcohol groups substituted with functional groups including aldehyde.

The reactive groups at both ends of the non-peptidyl linker may be the same or different. For example, one end of the non-peptidyl linker may have a maleimide group, and the other end may have an aldehyde group, a propionaldehyde group, or an alkyl aldehyde group such as butyl aldehyde. When a polyethylene glycol having hydroxyl reactive groups at both ends is used as the non-peptidyl linker, the hydroxy groups may be activated into various reactive groups by a known chemical reaction. Alternatively, a commercially available polyethylene glycol having a modified reactive group may be used to prepare the conjugate of the present disclosure.

As used herein, the term "physiologically active polypeptide" collectively refers to polypeptides having any physiological activity *in vivo,* which commonly have a polypeptide structure and have various physiological activities. The physiologically active polypeptides include those that function to regulate genetic expression and physiological function and to correct an abnormal condition caused by the lack or excessive secretion of a substance that is involved in the regulation of functions *in vivo.* The physiologically active polypeptides may also include general protein therapeutic agents. In addition, the term "physiologically active polypeptide" is meant to include not only native polypeptides, but also derivatives thereof.

The kind and size of physiologically active polypeptide in the conjugate of the present disclosure are not specifically limited, as long as it is a physiologically active polypeptide can show an increase in the serum half-life by the conjugate structure of the present disclosure. In an embodiment of the present disclosure, conjugates are prepared using various physiologically active polypeptides, including insulin, interferon, human growth hormone and a GLP-1 agonist, which are representative examples of physiologically active polypeptides, and it was found that the intrinsic binding affinity of the immunoglobulin Fc fragment itself for FcRn can be maintained regardless of the kind and size of physiologically active polypeptide.

The physiologically active polypeptide included in the conjugate according to the present disclosure may be selected from the group consisting of glucagon-like peptide-1 (GLP-1), granulocyte colony stimulating factor (G-CSF), human growth hormone (hGH), erythropoietin (EPO), glucagon, oxyntomodulin, insulin, growth hormone releasing hormone, growth hormone releasing peptide, interferons, interferon receptors, G-protein-coupled receptor, interleukins, interleukin receptors, enzymes, interleukin binding proteins, cytokine binding proteins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin, $\alpha$-lactalbumin, apolipoprotein-E, highly glycosylated erythropoietin, angiopoietins, hemoglobin, thrombin, thrombin receptor activating peptide, thrombomodulin, blood factors VII, VIIa, VIII, IX and XIII, plasminogen activating factor, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone stimulating protein, calcitonin, atriopeptin, cartilage inducing factor, elcatonin, connective tissue activating factor, tissue factor pathway inhibitor, follicle stimulating hormone, luteinizing hormone, luteinizing hormone releasing hormone, nerve growth factors, parathyroid hormone, relaxin, secretin, somatomedin, insulin-like growth factor, adrenocortical hormone, cholecystokinin, pancreatic polypeptide, gastrin releasing peptide, corticotropin releasing factor, thyroid stimulating hormone, autotaxin, lactoferrin, myostatin, cell surface antigens, virus derived vaccine antigens, monoclonal antibodies, polyclonal antibodies, and antibody fragments, but is not limited thereto. In addition, the term "physiologically active polypeptide", as used herein, is meant to include not only natural physiologically active polypeptides, but also agonists, precursors, derivatives, fragments or variants of each polypeptide. Herein, examples of oxyntomodulin derivatives include all those disclosed in Korean Patent Application Publication No. 10-2012-0137271, and examples of insulin-releasing peptide derivatives include those disclosed in Korean Patent Application Publication No. 10-2009-0008151, but are not limited thereto.

As used herein, the term "immunoglobulin Fc fragment" refers to a protein that contains the heavy-chain constant region of an immunoglobulin, excluding the heavy-chain constant region 1 (CH1) and light-chain constant region 1 (CL1) of the immunoglobulin. The Fc fragment may include a hinge region in the heavy-chain constant region. In the present disclosure, the immunoglobulin Fc fragment preferably comprises a CH2 domain, a CH3 domain, or both, because the binding affinity of the immunoglobulin Fc fragment for FcRn should be maintained.

Also, the immunoglobulin Fc region in the present disclosure may be an extended Fc fragment that includes a portion or whole of the heavy-chain constant region 1 (CH1) and/or the light-chain constant region 1 (CL1), except for the heavy-chain and light-chain variable regions of the immunoglobulin, as long as it maintains its intrinsic binding affinity for FcRn, even when it is linked to the physiologically active polypeptide and the non-peptidyl linker.

Because immunoglobulin Fc fragment is a biodegradable polypeptide metabolized *in vivo,* it is safe for use as a drug carrier. Also, because the immunoglobulin Fc fragment has a molecular weight lower than the entire immunoglobulin molecule, it is beneficial in terms of the preparation, purification and yield of the conjugate. In addition, because the Fab region, which shows high non-homogeneity due to the difference in amino acid sequence between antibodies, is removed, the Fc fragment has a considerably increased homogeneity and a low potential to induce serum antigenicity.

In the present disclosure, the immunoglobulin Fc fragment includes not only a native amino acid sequence, but also a sequence mutant thereof. As used herein, the term "amino acid sequence mutant" refers to a sequence that is different from the native amino acid sequence due to a deletion, insertion, non-conservative or conservative substitution or combinations thereof of one or more amino acid residues. For example, in the case of IgG Fc, amino acid residues at positions 214 to 238, 297 to 299, 318 to 322 or 327 to 331, known to be important in binding, may be used as a suitable target for modification.

In addition, various mutants are also possible, including mutants having a deletion of a region capable of forming a disulfide bond, a deletion of several amino acid residues at the N-terminus of a native Fc, or an addition of methionine residue to the N-terminus of a native Fc. Furthermore, to eliminate effector functions, a complement-binding site, for example, a C1q-binding site, may be removed, and an antibody dependent cell mediated cytotoxicity (ADCC) site may also be removed. Techniques of preparing such sequence derivatives of the immunoglobulin Fc fragment are disclosed in International Patent Publication Nos. WO 97/34631 and WO 96/32478.

Amino acid exchanges in proteins and peptides, which do not generally alter the activity of molecules, are known in the art (H. Neurath, R. L. Hill, The Proteins, Academic Press, New York,197 9). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly, in both directions.

In some cases, the immunoglobulin Fc fragment may also be modified by, for example, phosphorylation, sulfation, acrylation, glycosylation, methylation, farnesylation, acetylation or amidation.

The above-described Fc mutants are mutants that show the same biological activity as that of the Fc fragment of the present disclosure, but have improved structural stability against heat, pH, or the like.

In addition, these Fc fragments may be obtained from native forms isolated from humans and animals including cows, goats, pigs, mice, rabbits, hamsters, rats and guinea pigs, or may be recombinant forms or derivatives thereof, obtained from transformed animal cells or microorganisms. Herein, they may be obtained from a native immunoglobulin by isolating a whole immunoglobulin from the living body of humans or animals and treating it with protease. When the whole immunoglobulin is treated with papain, it is cleaved into Fab and Fc. Meanwhile, when it is treated with pepsin, it is cleaved into pF'c and $F(ab)_2$. These fragments may be subjected, for example, to size-exclusion chromatography to isolate Fc or pF'c.

Preferably, it is a recombinant immunoglobulin Fc fragment obtained from a microorganism using a human Fc fragment.

In addition, the immunoglobulin Fc fragment may be in the form of having native sugar chains, increased sugar chains compared to a native form or decreased sugar chains compared to the native form, or may be in a deglycosylated form. The increase, decrease or removal of the immunoglobulin Fc sugar chains may be performed using conventional methods, such as a chemical method, an enzymatic method and a genetic engineering method using a microorganism. The immunoglobulin Fc fragment obtained by removing sugar chains from an Fc shows a sharp decrease in binding affinity for the complement (c1q) and a decrease or loss in antibody-dependent cell-mediated cytotoxicity or complement-dependent cytotoxicity, and thus does not induce unnecessary immune responses *in vivo.* In this regard, an immunoglobulin Fc fragment in a deglycosylated or aglycosylated form may be more suitable for use as a drug carrier.

As used herein, the term "deglycosylation" refers to an enzymatic removal of sugar moieties from an Fc fragment, and the term "aglycosylation" means an unglycosylated Fc fragment produced in prokaryotes, preferably in *E. coli.*

Meanwhile, the immunoglobulin Fc fragment may originate from humans or animals such as cattle, goats, pigs, mice, rabbits, hamsters, rats or guinea pigs. Preferably, it is of human origin. In addition, the immunoglobulin Fc fragment may be an Fc fragment that is derived from IgG, IgA, IgD, IgE and IgM, combinations thereof, or hybrids thereof. Preferably, it is derived from IgG or IgM, which is among the most abundant proteins in the human blood, and most preferably it is derived from IgG known to enhance the half-life of ligand-binding proteins.

The term "combination", as used herein, refers to a formation of linkage between a polypeptide encoding single-chain immunoglobulin Fc fragments of the same origin and a single-chain polypeptide of a different origin to form a dimer or multimer. In other words, a dimer or multimer may be formed from two or more fragments selected from the group consisting of IgG Fc, IgA Fc, IgM Fc, IgD Fc, and IgE Fc fragments.

As used herein, the term "hybrid" refers to the presence of two or more sequences corresponding to immunoglobulin Fc fragments of different origins in a single-chain immunoglobulin Fc fragment. In the present disclosure, various types of hybrids are possible. In other words, domain hybrids may be composed of one to four domains selected from the group consisting of CH1, CH2, CH3 and CH4 of IgG Fc, IgM Fc, IgA Fc, IgE Fc and IgD Fc, and may include a hinge region.

On the other hand, IgG can be divided into IgG1, IgG2, IgG3 and IgG4 subclasses, and combinations or hybrids thereof may be used in the present disclosure, preferably IgG2 and IgG4 subclasses, and most preferably the Fc fragment of IgG4 rarely having effector functions such as CDC (complement dependent cytotoxicity). In other words, the most preferable immunoglobulin Fc fragment for use as a drug carrier in the present disclosure is a human IgG4-derived unglycosylated Fc fragment. The human Fc fragment is more preferable than a non-human Fc fragment, which may act as an antigen in the human body and cause undesirable immune responses such as the production of a new antibody against the antigen.

In an embodiment of the present disclosure, each of insulin, interferon, human growth hormone and a GLP-1 agonist is linked via a non-peptidyl linker to an immunoglobulin Fc fragment having the ability to bind to FcRn, thereby preparing conjugates that increase the intrinsic binding affinity of the immunoglobulin Fc fragment for FcRn and can easily dissociate from FcRn at a neutral pH and can also show a dissociation degree similar to that of the immunoglobulin Fc fragment (Examples and FIGS. 2 and 3). In addition, it was found that the conjugate of the present disclosure has a long *in vivo* duration of action compared to an in-frame conjugate (FIG. 4).

In still another aspect, the present disclosure provides a method for preparing a physiologically active polypeptide-immunoglobulin Fc fragment conjugate that maintains the intrinsic binding affinity of an immunoglobulin Fc fragment for FcRn, the method comprising the steps of: (a) linking a physiologically active polypeptide via a non-peptidyl linker to an immunoglobulin Fc fragment having an FcRn-binding region to prepare a mixture of physiologically active polypeptide-immunoglobulin Fc fragment conjugates; and (b) separating from the mixture a physiologically active polypeptide-immunoglobulin Fc fragment conjugate that shows a binding ratio within the range of ± 6% of the binding ratio of the immunoglobulin Fc fragment, as determined by substituting the amount bound to FcRn at pH 6.0 and the amount bound to FcRn at pH 7.4 into the following equation 1.

<u>Equation 1</u>

$$\text{Binding ratio (\%)} = (\text{amount bound at pH 7.4}/\ \text{amount bound at pH 6.0}) \times 100$$

Herein, the physiologically active polypeptide, the immunoglobulin Fc fragment, the non-peptidyl linker, the conjugate and the determination of the binding ratio are as described above.

Step (a) in the method of the present disclosure is a step of covalently linking a physiologically active polypeptide via a non-peptidyl linker to an immunoglobulin Fc fragment. Step (a) may comprise the steps of: (i) linking any one of the physiologically active polypeptide and the immunoglobulin Fc fragment to a reactive group at one end of the non-peptidyl linker; and (ii) linking the remaining one to a reactive group at the other end of the non-peptidyl linker. Step (a) may further comprise, between steps (i) and (ii), a step of separating the physiologically active polypeptide or immunoglobulin Fc fragment linked to one end of the non-peptidyl linker..

In order to link the physiologically active polypeptide via the non-peptidyl linker to a portion excluding the FcRn-binding region of the immunoglobulin Fc fragment, the physiologically active polypeptide having the non-peptidyl linker linked thereto may be reacted with the immunoglobulin Fc fragment at a pH of 4.0-9.0.

When the conjugate is prepared by this process, byproducts such as a conjugate showing a decrease in the binding affinity of the immunoglobulin Fc fragment for FcRn can be generated in addition to a conjugate that maintains the intrinsic binding affinity of the immunoglobulin Fc fragment for FcRn. For this reason, after the reaction that links the physiologically active polypeptide via the non-peptidyl peptide to the immunoglobulin Fc fragment, a process is additionally required to separate from the conjugate mixture a physiologically active polypeptide-immunoglobulin Fc fragment that maintains the intrinsic binding affinity of the immunoglobulin Fc fragment for FcRn.

Thus, the method of the present disclosure comprises step (b) of separating from the conjugate mixture a physiologically active polypeptide-immunoglobulin Fc fragment conjugate that shows a binding ratio within the range of ± 6% of the binding ratio of the immunoglobulin Fc fragment, as determined by substituting the amount boundg to FcRn at pH 6.0 and the amount bound to FcRn at pH 7.4 into equation 1.

Step (b) is preferably a process for separating a conjugate in which the non-peptidyl linker is linked to amino acid residues away from the FcRn-binding region of the immunoglobulin Fc fragment, for example, a region corresponding to positions 252-257 and 307-311 of CH2 and positions 433-436 of CH2, numbered according to the cobat numbering system. Specifically, step (b) is a process for selectively separating only a conjugate in which the non-peptidyl linker is connected to the N-terminus of the immunoglobulin Fc fragment so that the intrinsic binding affinity of the Fc fragment for FcRn is maintained.

Separation and purification conditions in step (b) may vary depending on the kinds of non-peptidyl linker, physiologically active polypeptide and the like used.

In still another aspect, the present disclosure provides a method of maintaining the intrinsic binding affinity of a physiologically active polypeptide-immunoglobulin Fc fragment conjugate for FcRn, the method comprising linking a

physiologically active polypeptide via a non-peptidyl linker to an immunoglobulin Fc fragment comprising an FcRn-binding region.

Herein, the physiologically active polypeptide, the immunoglobulin Fc fragment, the non-peptidyl linker and the conjugate are as described above.

The present disclosure has advantages in that, because the physiologically active polypeptide is linked via the non-peptidyl linker to the immunoglobulin Fc fragment comprising the FcRn-binding region, the intrinsic binding affinity of the immunoglobulin Fc fragment for FcRn is maintained while the immunoglobulin Fc fragment can easily dissociate from FcRn at a neutral pH and can be easily recycled, suggesting that the in *vivo* half-life of the immunoglobulin Fc fragment can be effectively increased.

Herein, the maintaining of the intrinsic binding affinity may be effected *in vivo.*

In still another aspect, the present disclosure provides a composition comprising the physiologically active polypeptide-immunoglobulin Fc fragment conjugate, which maintains the intrinsic binding affinity of the immunoglobulin Fc fragment for FcRn.

Herein, the physiologically active polypeptide, the immunoglobulin Fc fragment and the conjugate are as described above.

Hereinafter, the present disclosure will be described in further detail with reference to examples. It is to be understood, however, that these examples are for illustrative purposes only and are not intended to limit the scope of the present disclosure.

## Examples: Preparation of conjugates of physiologically active protein and Fc fragment comprising FcRn-binding site

### (1) Preparation of immunoglobulin Fc fragment

An immunoglobulin Fc fragment was prepared according to the method disclosed in Korean Patent Application No. 10-2006-0077377 (entitled "method for mass production of methionine residue-free immunoglobulin Fc region") filed in the name of the present authors.

### (2) Preparation of insulin-immunoglobulin Fc fragment conjugate

A reaction was performed to PEGylate a 3.4-kDa propion-ALD2 PEG (IDB, Korea) specifically at the N-terminus of the beta-chain of insulin. The reaction solution was purified using a cation-exchange column. To prepare an insulin-immunoglobulin Fc fragment conjugate, the purified mono-PEGylated insulin was reacted with the immunoglobulin Fc. At this time, the reaction was performed at pH 6.0-8.2 in order to direct the insulin specifically to the N-terminus of the immunoglobulin Fc. After completion of the reaction, the reaction solution was first purified using an anion-exchange column, and then purified using a hydrophobic column, thereby obtaining an insulin conjugate comprising insulin linked site-specifically to the immunoglobulin.

### (3) Preparation of interferon-immunoglobulin Fc fragment conjugate

A 3.4-kDa propion-ALD2 PEG (IDB, Korea) was added to and reacted with a buffer containing human interferon alpha-2b (hIFN$\alpha$-2b; molecular weight: 19 kDa) dissolved therein. To obtain a conjugate in which PEG is linked specifically to the amino terminal end of interferon-alpha and PEG and interferon-alpha are linked to each other at a ratio of 1:1, the reaction mixture was subjected to anion exchange column chromatography to purify a mono-PEGylated IFN$\alpha$-2b. To direct the purified mono-PEGylated interferon specifically to the N-terminus of the immunoglobulin Fc, a reaction was performed at a pH of 5.5-6.5. After the linking reaction, to purify the produced interferon-immunoglobulin Fc fragment conjugate, the reaction mixture was passed through an anion-exchange column to obtain an interferon-immunoglobulin Fc fragment conjugate fraction. The conjugate fraction was additionally purified using a hydrophobic column, thereby obtaining an interferon conjugate comprising interferon linked site-specifically to the immunoglobulin Fc.

### (4) Preparation of human growth hormone-immunoglobulin Fc fragment conjugate

A 3.4-kDa propion-ALD2 PEG (IDB, Korea) was added to and reacted with a buffer containing human growth hormone (hGH; molecular weight: 22 kDa) dissolved therein. To obtain a conjugate in which PEG is linked specifically to the amino terminal end of human growth hormone and PEG and human growth hormone are linked to each other at a ratio of 1:1, the reaction mixture was subjected to anion exchange column chromatography to purify a mono-PEGylated human growth hormone (hGH). To direct and link the purified mono-PEGylated human growth hormone specifically to the N-terminus of the immunoglobulin Fc, a reaction was performed at a pH of 5.5-6.5. After the linking reaction, the reaction mixture was purified using an anion-exchange column, thereby obtaining a human growth hormone conjugate

fraction comprising hormone growth hormone linked site-specifically to the immunoglobulin Fc.

(5) Preparation of GLP-1R agonist-immunoglobulin Fc fragment conjugate

A 3.4-kDa propion-ALD2 PEG (IDB, Korea) was reacted site-specifically with the lysine residue of imidazo-acetyl-exendin-4 (CA exendin-4, Bachem, Switzerland). To obtain a conjugate in which PEG and the GLP-1R agonist are linked to each other at a ratio of 1:1, the reaction mixture was then subjected to cation-exchange column chromatography to purify mono-PEGylated exendin-4. To prepare a GLP-1R agonist-immunoglobulin Fc fragment conjugate comprising the mono-PEGylated exendin-4 linked specifically to the N-terminus of the immunoglobulin Fc, a reaction was performed at a pH of 5.0-8.2. After the coupling reaction, a two-step purification process was performed using a hydrophobic column and an anion-exchange column, thereby obtaining a GLP-1R agonist-immunoglobulin Fc fragment conjugate comprising the GLP-1R agonist linked site-specifically to the immunoglobulin Fc.

Comparative Example: Preparation of conjugate comprising GLP-1R agonist linked in-frame to immunoglobulin fragment

For comparison with the conjugate of the present disclosure, a fusion protein of a GLP-1R agonist with an immunoglobulin Fc fragment comprising an about 50 kDa FcRn site was prepared by a gene recombination method without using a linker. The conjugate comprising the GLP-1R agonist linked in-frame to the immunoglobulin Fc fragment (hereinafter also referred to as "in-frame GLP-1R agonist-immunoglobulin Fc fragment conjugate") was purified from the culture using an affinity column.

Experimental Example 1: Evaluation of binding affinity for FcRn

Among proteins introduced into cells by endocytosis, a protein having an FcRn-binding site binds to FcRn at an acidic pH, whereas a protein that does not bind to FcRn is removed by lysosomal degradation. When the protein bound to FcRn dissociates from FcRn at a neutral pH, it is released to the cell surface, whereas the FcRn-bound protein that does not dissociate from FcRn undergoes lysosomal degradation. This mechanism is shown in FIG. 1.

Thus, in order to examine whether the conjugate of the Example, obtained by linking the physiologically active protein via the non-peptidyl linker to the immunoglobulin Fc comprising the FcRn binding site, can maintain the binding affinity of the immunoglobulin Fc alone for FcRn or whether it can easily dissociate from FcRn at a neutral pH and can be released into blood, the following experiment was performed.

Specifically, in order to examine whether the binding affinity of the immunoglobulin fragment for FcRn does not change even when the conjugate of the present disclosure is formed, the binding affinity between FcRn and the conjugate of the Example or the Comparative Example was measured using SPR (surface Plasmon resonance, BIACORE 3000). As FcRn, FCGRT & B2M dimer receptor (Sino Biological Inc.) was used. FcRn was immobilized onto a CM5 chip using an amine coupling method, and then the conjugate was added thereto at a concentration between 100 nM to 12.5 nM to measure the binding affinity therebetween. However, because the mechanism of the FcRn-mediated increase in the half-life depends on pH, the experiment was carried out at each of an acidic pH and a neutral pH.

(1) Measurement of the binding affinity of immunoglobulin-protein conjugate for FcRn at acidic pH

Because the binding of an endocytosed protein to FcRn occurs at an acidic pH, a phosphate buffer (pH 6.0) was used as running buffer-1 in order to reproduce this binding. All the immunoglobulin fragment-protein conjugates were diluted in running buffer-1 to induce the binding, and dissociation of the conjugates was also performed using running buffer-1. Each of the immunoglobulin fragment-protein conjugates was brought contact with the FcRn-immobilized chip for 4 minutes to induce the binding thereof, and then subjected to a dissociation process for 6 minutes. Next, in order to measure the values of the immunoglobulin fragment-protein conjugates at different concentrations, i.e., binds the immunoglobulin fragment-protein conjugates at different concentrations to the chip, pH 7.4 Hepes buffer was brought into contact with the immunoglobulin fragment-protein conjugates bound to FcRn for about 30 seconds. The binding affinity of each of the immunoglobulin fragment-protein conjugates for FcRn at an acidic pH was analyzed using the 1:1 Langmuir binding with drifting baseline model in the BIAevaluation software, and the results of the analysis are shown in FIG. 2.

(2) Measurement of the binding affinity of immunoglobulin-protein conjugate for FcRn at neutral pH

Because the release of the conjugate from cells after binding to FcRn occurs when the pH changes from an acidic pH to a neutral pH, Hepes buffer (pH 7.4) was used as running buffer-2. However, the binding between FcRn and each of the immunoglobulin fragment-protein conjugates was induced for 4 minutes using the sample comprising each

of the immunoglobulin fragment-protein conjugates, which diluted in running buffer-1, and then dissociation of each of the immunoglobulin fragment-protein conjugates from FcRn was induced for 1 minute using running buffer-2. Sensorgrams of the immunoglobulin fragment-protein conjugates are shown in FIG. 3. The degree of dissociation of the immunoglobulin fragment-protein conjugate from FcRn was expressed as a binding ratio (%) according to the following equation 2. Herein, the resonance unit measured at 2 seconds before the completion of injection of the pH 6.0 sample was selected as a physical amount that is proportional to the amount bound at pH 6.0, and the resonance unit measured at 10 seconds after the start of dissociation at pH 7.4 was selected as a physical amount that is proportional to the amount bound at pH 7.4. Using the selected resonance units, the binding ratio was calculated according to the following equation 2:

Equation 2

$$\text{Binding ratio (\%)} = (\text{amount bound at pH 7.4/ amount bound at pH 6.0}) \times 100$$

As used herein, the term "binding ratio" refers to the extent to which the immunoglobulin fragment-protein conjugate easily dissociates from FcRn. It can be seen that, as the value of the binding ratio becomes smaller, the dissociation of the conjugate at a neutral pH is better and the FcRn-mediated recycling of the conjugate more easily occurs, whereas, as the value of the binding ratio becomes larger, the dissociation of the conjugate at a neutral pH is insufficient, so that the conjugate is more likely to be removed by lysosomal degradation even when it is endocytosed by binding to FcRn.

As a result, as shown in FIGS. 2 and 3, the various immunoglobulin fragment-protein conjugates of the Example did bind to FcRn in a concentration-dependent manner. The results are also shown in Table 1 below, and the degrees of dissociation of the conjugates, calculated using equation 2, are shown in Table 2 below.

Table 1: Comparison of binding affinities of immunoglobulin fragment-protein conjugates for FcRn at pH 6.0

| Test materials | pH 6.0 | | |
|---|---|---|---|
| | ka (1/Ms, X$10^5$) | kd (1/s, X$10^{-3}$) | $K_D$ (nM) |
| Immunoglobulin fragment | 4.8 | 10.5 | 22.0±3.0 |
| Insulin-immunoglobulin fragment conjugate | 3.1 | 7.1 | 22.6±3.9 |
| Interferon-immunoglobulin fragment conjugate | 3.0 | 9.3 | 30.0±4.8 |
| Human growth hormone-immunoglobulin fragment conjugate | 1.2 | 3.6 | 26.8±9.0 |
| GLP-1R agonist-immunoglobulin fragment conjugate | 3.8 | 9.5 | 24.7±5.5 |
| In-frame GLP-1R agonist-immunoglobulin fragment conjugate | 3.2 | 5.3 | 17.0±3.7 |
| *ka: association rate constant, kd: dissociation rate constant, $K_D$: affinity constant, binding ratio: a value obtained by dividing the amount of binding at pH 7.4 by the amount of binding at pH 6.0 and multiplying the divided value by 100. | | | |

Table 2: Comparison of degrees of dissociation of immunoglobulin fragment-protein conjugates from FcRn

| Test materials | Concentration (nM) | Bindingamount (RU) at pH6.0 | Bindingamount (RU) at pH7.4 | Binding ratio (%) | Average binding ratio (%) |
|---|---|---|---|---|---|
| Immunoglobulin fragment | 100 | 163.7 | 4.8 | 2.9 | 5.4±2.0 |
| | 50 | 137.4 | 6.5 | 4.7 | |
| | 25 | 110.9 | 7.4 | 6.6 | |
| | 12.5 | 88.0 | 6.5 | 7.3 | |

(continued)

| Test materials | Concentration (nM) | Bindingamount (RU) at pH6.0 | Bindingamount (RU) at pH7.4 | Binding ratio (%) | Average binding ratio (%) |
|---|---|---|---|---|---|
| Insulin-immunoglobulin fragment conjugate | 100 | 214.1 | 4.8 | 2.2 | 5.0±2.2 |
| | 50 | 174.3 | 8.0 | 4.6 | |
| | 25 | 143.9 | 8.4 | 5.9 | |
| | 12.5 | 106.6 | 7.9 | 7.4 | |
| Interferon-immunoglobulin fragment conjugate | 100 | 196.9 | 6.4 | 3.2 | 5.0±1.6 |
| | 50 | 157.2 | 6.7 | 4.3 | |
| | 25 | 120.3 | 6.7 | 5.6 | |
| | 12.5 | 91.3 | 6.4 | 7.0 | |
| Human growth hormone-immunoglobulin fragment conjugate | 100 | 205.7 | 8.2 | 4.0 | 6.6±2.6 |
| | 50 | 153.0 | 7.7 | 5.1 | |
| | 25 | 111.9 | 8.3 | 7.4 | |
| | 12.5 | 82.9 | 8.1 | 9.8 | |
| GLP-1R agonist-immunoglobulin fragment conjugate | 100 | 163.5 | 6.2 | 3.8 | 6.6 ± 2.6 |
| | 50 | 135.5 | 8.1 | 5.9 | |
| | 25 | 107.7 | 7.2 | 6.7 | |
| | 12.5 | 86.2 | 8.6 | 10.0 | |
| In-frame GLP-1R agonist-immunoglobulin fragment conjugate | 100 | 301.7 | 38.2 | 12.7 | 12.7±0.3 |
| | 50 | 249.5 | 31.2 | 12.5 | |
| | 25 | 197.6 | 25.9 | 13.1 | |
| | 12.5 | 148.7 | 18.6 | 12.5 | |

As can be seen in Tables 1 and 2 above, the binding affinity at an acidic pH or a neutral pH did not significantly differ between the immunoglobulin fragment alone (an independent Fc having no therapeutic protein linked thereto) and the conjugates of the present disclosure. In other words, it was found that, in the case of the immunoglobulin-protein conjugates produced according to the present disclosure, the binding affinity of the immunoglobulin fragment for FcRn did not change even when the immunoglobulin did bind to the physiologically active protein, and particularly, the conjugates comprising various physiologically active proteins having different sizes showed similar results. However, the in-frame GLP-1R agonist-immunoglobulin Fc fragment conjugate of the Comparative Example showed a high binding ratio, and thus could not suitably dissociate from FcRn at a neutral pH, suggesting that it has a lower effect on prolonging the half-life of the physiologically active protein, compared to the conjugate of the present disclosure.

Experimental Example 2: Test for comparison of *in vivo* pharmacokinetics between GLP-1R agonist-immunoglobulin Fc fragment conjugate

In order to compare *in vivo* pharmacokinetics between the GLP-1R agonist-immunoglobulin Fc fragment conjugate of Example (5) and the in-frame GLP-1R agonist-immunoglobulin Fc fragment conjugate, changes in the serum concentrations of the conjugates were analyzed using normal SD rats.

Specifically, each of the GLP-1R agonist-immunoglobulin Fc fragment conjugate (400 mcg/kg) and the in-frame GLP-1R agonist-immunoglobulin Fc fragment conjugate (400 mcg/kg) was diluted in physiological saline and administered subcutaneously to the animals at a dose of 2 mL/kg. At 4, 8, 24, 48, 72, 96, 120, 144, 168, 192, 216, 240, 288, 312 and 336 hours after administration of the test materials, blood was collected from the jugular vein of the rats, and serum was separated from the blood. Next, the concentration of the drug in each of the serum samples was quantified by an enzyme-linked immunosorbent assay, and the results of the quantification are shown in FIG. 4.

As a result, the serum half-lives of the GLP-1R agonist-immunoglobulin Fc fragment conjugate and the in-frame GLP-1R agonist-immunoglobulin Fc fragment conjugate were 40.9 hours and 28 hours, respectively, and the maximum serum concentrations of the conjugates were 1758.6 ng/mL and 742.7 ng/mL, respectively. In other words, when the drugs were administered subcutaneously to the normal rats at the same dose, it was shown that the GLP-1R agonist-immunoglobulin fragment conjugate of the present disclosure was excellent in terms of the *in vivo* absorption and half-life compared to the in-frame GLP-1R agonist-immunoglobulin Fc fragment conjugate (FIG. 4).

## Claims

1. A composition, comprising a physiologically active polypeptide-immunoglobulin Fc fragment conjugate which maintains the intrinsic binding affinity of the immunoglobulin Fc fragment for FcRn,
the conjugate comprising only one molecule of a physiologically active polypeptide linked via a non-peptidyl linker to an immunoglobulin Fc fragment comprising an FcRn-binding region,
wherein a binding ratio of the conjugate is within the range of ± 6% of the binding ratio of the immunoglobulin Fc fragment determined under the same conditions as those used for the conjugate, wherein the binding ratio of the conjugate and the binding ratio of the immunoglobulin Fc fragment are determined using the following equation:

Binding ratio (%) = (amount bound to FcRn at pH 7.4/ amount bound to FcRn at pH 6.0) X 100;

wherein the composition comprises only monomeric forms of said conjugate; and
wherein the physiologically active polypeptide is selected from the group consisting of glucagon-like peptide-1 (GLP-1), GLP-1 receptor agonist, granulocyte colony stimulating factor (G-CSF), human growth hormone (hGH), erythropoietin (EPO), glucagon, oxyntomodulin, insulin, growth hormone releasing hormone, growth hormone releasing peptide, interferons, interferon receptors, G-protein-coupled receptor, interleukins, interleukin receptors, enzymes, interleukin binding proteins, cytokine binding proteins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin, α-lactalbumin, apolipoprotein-E, highly glycosylated erythropoietin, angiopoietins, hemoglobin, thrombin, thrombin receptor activating peptide, thrombomodulin, blood factors VII, VIIa, VIII, IX and XIII, plasminogen activating factor, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone stimulating protein, calcitonin, atriopeptin, cartilage inducing factor, elcatonin, connective tissue activating factor, tissue factor pathway inhibitor, follicle stimulating hormone, luteinizing hormone, luteinizing hormone releasing hormone, nerve growth factors, parathyroid hormone, relaxin, secretin, somatomedin, insulin-like growth factor, adrenocortical hormone, cholecystokinin, pancreatic polypeptide, gastrin releasing peptide, corticotropin releasing factor, thyroid stimulating hormone, autotaxin, lactoferrin, myostatin, cell surface antigens, virus derived vaccine antigens, monoclonal antibodies, polyclonal antibodies, and antibody fragments.

2. The composition of claim 1, wherein the non-peptidyl linker is selected from the group consisting of polyethylene glycol, polypropylene glycol, an ethylene glycol-propylene glycol copolymer, polyoxyethylated polyol, polyvinyl alcohol, polysaccharide, dextran, polyvinyl ethyl ether, a biodegradable polymer, a lipid polymer, chitin, hyaluronic acid, and combinations thereof.

3. The composition of claim 1, wherein the non-peptidyl linker is a polyethylene glycol polymer represented by the following formula:

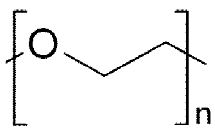

wherein n ranges from 10 to 2400.

4. The composition of claim 1, wherein the non-peptidyl linker has a reactive group selected from the group consisting

of an aldehyde group, a propionaldehyde group, a butyraldehyde group, a maleimide group, and succinimide derivatives.

**5.** The composition of claim 1, wherein the physiologically active polypeptide is selected from the group consisting of insulin, interferons, human growth hormone (hGH), and GLP-1 receptor agonist.

**6.** The composition of claim 1, wherein the immunoglobulin Fc fragment comprising the FcRn-binding region comprises a CH2 domain, a CH3 domain, or both.

**7.** The composition of claim 1, wherein the immunoglobulin Fc fragment is in a non-glycosylated form.

**8.** The composition of claim 1, wherein the immunoglobulin Fc fragment further comprises a hinge region.

**9.** The composition of claim 1, wherein the immunoglobulin Fc fragment is a Fc fragment derived from the group consisting of IgG, IgA, IgD, IgE, IgM, combinations thereof, and hybrids thereof.

**10.** The composition of claim 1, wherein the immunoglobulin Fc fragment is an IgG4 Fc fragment.

**11.** A method for preparing a composition according to claim 1, the method comprising:

(a) linking a physiologically active polypeptide via a non-peptidyl linker to an immunoglobulin Fc fragment comprising an FcRn-binding region to prepare a mixture of physiologically active polypeptide-immunoglobulin Fc fragment conjugates; and
(b) separating from the mixture physiologically active polypeptide-immunoglobulin Fc fragment conjugates that comprise only one molecule of a physiologically active polypeptide linked via a non-peptidyl linker to an immunoglobulin Fc fragment comprising an FcRn-binding region and which show a binding ratio within the range of ± 6% of the binding ratio of the immunoglobulin Fc fragment, determined under the same conditions as those used for the conjugate, wherein the binding ratio of the conjugate and the binding ratio of the immunoglobulin Fc fragment are determined using the following equation:

$$\text{Binding ratio (\%)} = (\text{amount bound to FcRn at pH 7.4/ amount bound to FcRn at pH 6.0}) \times 100.$$

**12.** The method of claim 11, wherein the non-peptidyl linker is a polyethylene glycol polymer represented by the following formula:

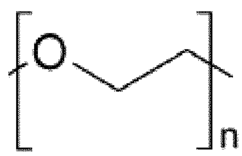

wherein n ranges from 10 to 2400.

**13.** The method of claim 11, wherein the conjugate separated in step (b) has a structure in which the non-peptidyl linker is bound to the N-terminus of the immunoglobulin Fc fragment.

**14.** The method of claim 11, wherein step (b) comprises determining a binding ratio of the conjugate and a binding ratio of the immunoglobulin Fc fragment using the equation before separating the physiologically active polypeptide-immunoglobulin Fc fragment conjugate.

**15.** The method of claim 11, wherein the physiologically active polypeptide is selected from the group consisting of insulin, interferons, human growth hormone (hGH), and GLP-1 receptor agonist

**16.** The method of claim 11, wherein

(a) the immunoglobulin Fc fragment comprising the FcRn-binding region comprises a CH2 domain, a CH3

domain, or both;
(b) the immunoglobulin Fc fragment is in a non-glycosylated form;
(c) the immunoglobulin Fc fragment further comprises a hinge region;
(d) the immunoglobulin Fc fragment is a Fc fragment derived from the group consisting of IgG, IgA, IgD, IgE, IgM, combinations thereof, and hybrids thereof; or
(e) the immunoglobulin Fc fragment is an IgG4 Fc fragment.

**Patentansprüche**

1. Zusammensetzung, umfassend ein Konjugat aus physiologisch aktivem Polypeptid und Immunglobulin-Fc-Fragment, das die intrinsische Bindungsaffinität des Immunglobulin-Fc-Fragments zu FcRn beibehält,
wobei das Konjugat nur ein Molekül eines physiologisch aktiven Polypeptids umfasst, das über einen Nichtpeptidyllinker an ein Immunglobulin-Fc-Fragment gebunden ist, das eine FcRn-Bindungsregion umfasst,
wobei ein Bindungsverhältnis des Konjugats im Bereich von ± 6 % des Bindungsverhältnisses des Immunglobulin-Fc-Fragments liegt, das unter denselben Bedingungen wie den für das Konjugat verwendeten bestimmt wird, wobei das Bindungsverhältnis des Konjugats und das Bindungsverhältnis des Immunglobulin-Fc-Fragments unter Verwendung der folgenden Gleichung bestimmt werden:

Bindungsverhältnis (%) = (bei pH 7,4 an FcRn gebundene Menge / bei pH 6,0 an FcRn gebundene Menge) x 100;

wobei die Zusammensetzung nur monomere Formen des Konjugats umfasst; und
wobei das physiologisch aktive Polypeptid ausgewählt ist aus der Gruppe bestehend aus Glucagon-ähnlichem Peptid-1 (GLP-1), GLP-1-Rezeptoragonist, Granulozyten-Koloniestimulierendem Faktor (G-CSF), menschlichem Wachstumshormon (hGH), Erythropoietin (EPO), Glucagon, Oxyntomodulin, Insulin, Wachstumshormon freisetzendem Hormon, Wachstumshormon freisetzendem Peptid, Interferonen, Interferonrezeptoren, G-Protein-gekoppeltem Rezeptor, Interleukinen, Interleukinrezeptoren, Enzymen, Interleukin bindenden Proteinen, Zytokin bindenden Proteinen, Makrophagen aktivierendem Faktor, Makrophagenpeptid, B-Zellfaktor, T-Zellfaktor, Protein A, Allergiehemmer, Zellnekrose-Glykoproteinen, Immuntoxin, Lymphotoxin, Tumornekrosefaktor, Tumorsuppressoren, Metastasewachstumsfaktor, α-1-Antitrypsin, Albumin, α-Lactalbumin, Apolipoprotein-E, stark glykosyliertem Erythropoietin, Angiopoetinen, Hämoglobin, Thrombin, Thrombinrezeptor aktivierendem Peptid, Thrombomodulin, Blutfaktoren VII, VIIa, VIII, IX und XIII, Plasminogen aktivierendem Faktor, Fibrin bindendem Peptid, Urokinase, Streptokinase, Hirudin, Protein C, C-reaktivem Protein, Reninhemmer, Collagenasehemmer, Superoxiddismutase, Leptin, Blutplättchen-Wachstumsfaktor, epithelialem Wachstumsfaktor, epidermalem Wachstumsfaktor, Angiostatin, Angiotensin, Knochenwachstumsfaktor, knochenstimulierendem Protein, Calcitonin, Atriopeptin, knorpelinduzierendem Faktor, Elcatonin, Bindegewebe aktivierendem Faktor, Gewebefaktorweghemmer, follikelstimulierendem Hormon, luteinisierendem Hormon, luteinisierendes Hormon freisetzendem Hormon, Nervenwachstumsfaktoren, Parathormon, Relaxin, Sekretin, Somatomedin, insulinähnlichem Wachstumsfaktor, Nebennierenrindenhormon, Cholecystokinin, pankreatischem Polypeptid, Gastrin freisetzendem Peptid, Corticotropin freisetzendem Faktor, thyreotropem Hormon, Autotaxin, Laktoferrin, Myostatin, Zelloberflächenantigenen, Virusvakzineantigenen, monoklonalen Antikörpern, polyklonalen Antikörpern und Antikörperfragmenten.

2. Zusammensetzung nach Anspruch 1, wobei der Nichtpeptidyllinker aus der aus Polyethylenglykol, Polypropylenglykol, einem Ethylenglykol-Propylenglykol-Copolymer, polyoxyethyliertem Polyol, Polyvinylalkohol, Polysaccharid, Dextran, Polyvinylethylether, einem biologisch abbaubaren Polymer, einem Lipidpolymer, Chitin, Hyaluronsäure und Kombinationen davon bestehenden Gruppe ausgewählt ist.

3. Zusammensetzung nach Anspruch 1, wobei der Nichtpeptidyllinker ein Polyethylenglykolpolymer ist, das durch die nachstehende Formel dargestellt ist:

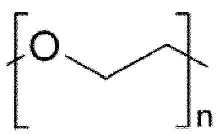

worin n im Bereich von 10 bis 2.400 liegt.

4. Zusammensetzung nach Anspruch 1, wobei der Nichtpeptidyllinker eine reaktive Gruppe aufweist, die aus der aus einer Aldehydgruppe, einer Propionaldehydgruppe, einer Butyraldehydgruppe, einer Maleinimidgruppe und Succinimidderivaten bestehenden Gruppe ausgewählt ist.

5. Zusammensetzung nach Anspruch 1, wobei das physiologisch aktive Polypeptid aus der aus Insulin, Interferonen, menschlichem Wachstumshormon (hGH) und GLP-1-Rezeptor-agonist bestehenden Gruppe ausgewählt ist.

6. Zusammensetzung nach Anspruch 1, wobei das Immunglobulin-Fc-Fragment, das die FcRn-Bindungsregion umfasst, eine CH2-Domäne, eine CH3-Domäne oder beides umfasst.

7. Zusammensetzung nach Anspruch 1, wobei das Immunglobulin-Fc-Fragment in einer nichtglykosylierten Form vorliegt.

8. Zusammensetzung nach Anspruch 1, wobei das Immunglobulin-Fc-Fragment weiters eine Gelenkregion umfasst.

9. Zusammensetzung nach Anspruch 1, wobei das Immunglobulin-Fc-Fragment ein Fc-Fragment ist, das aus der aus IgG, IgA, IgD, IgE, IgM, Kombinationen davon und Hybriden davon bestehenden Gruppe ausgewählt ist.

10. Zusammensetzung nach Anspruch 1, wobei das Immunglobulin-Fc-Fragment ein IgG4-Fc-Fragment ist.

11. Verfahren zur Herstellung einer Zusammensetzung nach Anspruch 1, wobei das Verfahren Folgendes umfasst:

(a) Binden eines physiologisch aktiven Polypeptids über einen Nichtpeptidyllinker an ein Immunglobulin-Fc-Fragment, das eine FcRn-Bindungsregion umfasst, um ein Gemisch aus Konjugaten aus physiologisch aktivem Polypeptid und Immunglobulin-Fc-Fragment herzustellen; und
(b) Abtrennen von Konjugaten aus physiologisch aktivem Polypeptid und Immunglobulin-Fc-Fragment aus dem Gemisch, die nur ein Molekül eines physiologisch aktiven Polypeptids umfassen, das über einen Nichtpeptidyllinker an ein Immunglobulin-Fc-Fragment gebunden ist, das eine FcRn-Bindungsregion umfasst, und die ein Bindungsverhältnis im Bereich von ± 6 % des Bindungsverhältnisses des Immunglobulin-Fc-Fragments zeigen, das unter denselben Bedingungen wie den für das Konjugat verwendeten bestimmt wird, wobei das Bindungsverhältnis des Konjugats und das Bindungsverhältnis des Immunglobulin-Fc-Fragments unter Verwendung der folgenden Gleichung bestimmt werden:

Bindungsverhältnis (%) = (bei pH 7,4 an FcRn gebundene Menge / bei pH 6,0 an FcRn gebundene Menge) x 100.

12. Verfahren nach Anspruch 11, wobei der Nichtpeptidyllinker ein Polyethylenglykolpolymer ist, das durch die nachstehende Formel dargestellt ist:

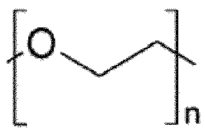

worin n im Bereich von 10 bis 2.400 liegt.

13. Verfahren nach Anspruch 11, wobei das in Schritt (b) abgetrennte Konjugat eine Struktur aufweist, in der der Nichtpeptidyllinker an den N-Terminus des Immunglobulin-Fc-Fragments gebunden ist.

14. Verfahren nach Anspruch 11, wobei Schritt (b) das Bestimmen eines Bindungsverhältnisses des Konjugats und eines Bindungsverhältnisses des Immunglobulin-Fc-Fragments unter Verwendung der Gleichung vor dem Abtrennen des Konjugats aus physiologisch aktivem Polypeptid und Immunglobulin-Fc-Fragment umfasst.

15. Verfahren nach Anspruch 11, wobei das physiologisch aktive Polypeptid aus der aus Insulin, Interferonen, menschlichem Wachstumshormon (hGH) und GLP-1-Rezeptoragonist bestehenden Gruppe ausgewählt ist.

**16.** Verfahren nach Anspruch 11, wobei

(a) das Immunglobulin-Fc-Fragment, das die FcRn-Bindungsregion umfasst, eine CH2-Domäne, eine CH3-Domäne oder beides umfasst;
(b) das Immunglobulin-Fc-Fragment in einer nichtglykosylierten Form vorliegt;
(c) das Immunglobulin-Fc-Fragment weiters eine Gelenkregion umfasst;
(d) das Immunglobulin-Fc-Fragment ein Fc-Fragment ist, das aus der aus IgG, IgA, IgD, IgE, IgM, Kombinationen davon und Hybriden davon bestehenden Gruppe stammt; oder
(e) das Immunglobulin-Fc-Fragment ein IgG4-Fc-Fragment ist.

**Revendications**

**1.** Composition, comprenant un conjugué polypeptide physiologiquement actif-fragment Fc d'immunoglobuline qui maintien l'affinité de liaison intrinsèque du fragment Fc d'immunoglobuline pour FcRn,
le conjugué comprenant uniquement une molécule d'un polypeptide physiologiquement actif lié par le biais d'un lieur non peptidylique à un fragment Fc d'immunoglobuline comprenant une région se liant à FcRn,
dans laquelle un rapport de liaison du conjugué est compris dans la plage de ±6 % du rapport de liaison du fragment Fc d'immunoglobuline déterminé dans les mêmes conditions que celles utilisées pour le conjugué, dans laquelle le rapport de liaison du conjugué et le rapport de liaison du fragment Fc d'immunoglobuline sont déterminés à l'aide de l'équation suivante :

Rapport de liaison (%) = (quantité liée à FcRn à pH 7,4/quantité liée à FcRn à pH 6,0) X 100 ;

dans laquelle la composition comprend uniquement des formes monomères dudit conjugué ; et
dans laquelle le polypeptide physiologiquement actif est choisi dans le groupe constitué par le glucagon-like peptide-1 (GLP-1), un agoniste des récepteurs de GLP-1, le facteur de stimulation des colonies de granulocytes (G-CSF), l'hormone de croissance humaine (hGH), l'érythropoïétine (EPO), le glucagon, l'oxyntomoduline, l'insuline, l'hormone de libération de l'hormone de croissance, le peptide de libération de l'hormone de croissance, les interférons, les récepteurs des interférons, le récepteur couplé aux protéines G, les interleukines, les récepteurs des interleukines, les enzymes, les protéines de liaison aux interleukines, les protéines de liaison à la cytokine, le facteur d'activation des macrophages, un peptide pour macrophages, le facteur des lymphocytes B, le facteur des lymphocytes T, la protéine A, un inhibiteur des allergies, les glycoprotéines de nécrose cellulaire, l'immunotoxine, la lymphotoxine, le facteur de nécrose tumorale, les suppresseurs de tumeurs, le facteur de croissance des métastases, l'alpha-1 antitrypsine, l'albumine, l'$\alpha$-lactalbumine, l'apolipoprotéine-E, l'érythropoïétine hautement glycosylée, les angiopoïétines, l'hémoglobine, la thrombine, le peptide activant le récepteur de la thrombine, la thrombomoduline, les facteurs sanguins VII, VIIa, VIII, IX et XIII, le facteur activant le plasminogène, un peptide se liant à la fibrine, l'urokinase, la streptokinase, l'hirudine, la protéine C, la protéine C réactive, un inhibiteur de la rénine, un inhibiteur de la collagénase, la superoxyde dismutase, la leptine, le facteur de croissance dérivé des plaquettes, le facteur de croissance épithélial, le facteur de croissance épidermique, l'angiostatine, l'angiotensine, le facteur de croissance osseuse, la protéine stimulant les os, la calcitonine, l'atriopeptine, le facteur induisant le cartilage, l'elcatonine, le facteur d'activation du tissu conjonctif, l'inhibiteur de la voie du facteur tissulaire, l'hormone folliculo-stimulante, l'hormone lutéinisante, l'hormone libérant l'hormone lutéinisante, les facteurs de croissance nerveuse, la parathormone, la relaxine, la sécrétine, la somatomédine, le facteur de croissance analogue à l'insuline, l'hormone corticosurrénale, la cholécystokinine, le polypeptide pancréatique, le peptide libérant la gastrine, le facteur libérant la corticotropine, l'hormone stimulant la thyroïde, l'autotaxine, la lactoferrine, la myostatine, les antigènes de surface cellulaire, les antigènes vaccinaux dérivés de virus, les anticorps monoclonaux, les anticorps polyclonaux et des fragments d'anticorps.

**2.** Composition selon la revendication 1, dans laquelle le lieur non peptidylique est choisi dans le groupe constitué par le polyéthylène glycol, le polypropylène glycol, un copolymère d'éthylène glycol-propylène glycol, un polyol polyoxyéthylé, un alcool polyvinylique, un polysaccharide, le dextrane, un éther polyvinyléthylique, un polymère biodégradable, un polymère lipidique, la chitine, l'acide hyaluronique, et des combinaisons de ceux-ci.

**3.** Composition selon la revendication 1, dans laquelle le lieur non peptidylique est un polymère de polyéthylène glycol

représenté par la formule suivante :

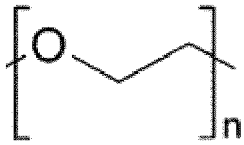

dans laquelle n est compris dans la plage allant de 10 à 2 400.

4. Composition selon la revendication 1, dans laquelle le lieur non peptidylique possède un groupe réactif choisi dans le groupe constitué par un groupe aldéhyde, un groupe propionaldéhyde, un groupe butyraldéhyde, un groupe maléimide et des dérivés succinimidiques.

5. Composition selon la revendication 1, dans laquelle le polypeptide physiologiquement actif est choisi dans le groupe constitué par l'insuline, les interférons, l'hormone de croissance humaine (hGH) et un agoniste des récepteurs de GLP-1.

6. Composition selon la revendication 1, dans laquelle le fragment Fc d'immunoglobuline comprenant la région se liant à FcRn comprend un domaine CH2, un domaine CH3, ou les deux.

7. Composition selon la revendication 1, dans laquelle le fragment Fc d'immunoglobuline est sous une forme non glycosylée.

8. Composition selon la revendication 1, dans laquelle le fragment Fc d'immunoglobuline comprend en outre une région charnière.

9. Composition selon la revendication 1, dans laquelle le fragment Fc d'immunoglobuline est un fragment Fc dérivé du groupe constitué par l'IgG, l'IgA, l'IgD, l'IgE, l'IgM, des combinaisons de celles-ci, et des hybrides de celles-ci.

10. Composition selon la revendication 1, dans laquelle le fragment Fc d'immunoglobuline est un fragment Fc de l'IgG4.

11. Procédé de préparation d'une composition selon la revendication 1, le procédé comprenant :

(a) la liaison d'un polypeptide physiologiquement actif par le biais d'un lieur non peptidylique à un fragment Fc d'immunoglobuline comprenant une région se liant à FcRn pour préparer un mélange de conjugués polypeptide physiologiquement actif-fragment Fc d'immunoglobuline ; et
(b) la séparation du mélange des conjugués polypeptide physiologiquement actif-fragment Fc d'immunoglobuline qui comprennent uniquement une molécule d'un polypeptide physiologiquement actif lié par le biais d'un lieur non peptidylique à un fragment Fc d'immunoglobuline comprenant une région se liant à FcRn et qui montre un rapport de liaison compris dans la plage de ±6 % du rapport de liaison du fragment Fc d'immunoglobuline, déterminé dans les mêmes conditions que celles utilisées pour le conjugué, dans laquelle le rapport de liaison du conjugué et le rapport de liaison du fragment Fc d'immunoglobuline sont déterminés à l'aide de l'équation suivante :

Rapport de liaison (%) = (quantité liée à FcRn à pH 7,4/quantité liée à FcRn à pH 6,0) X 100.

12. Procédé selon la revendication 11, dans lequel le lieur non peptidylique est un polymère de polyéthylène glycol représenté par la formule suivante :

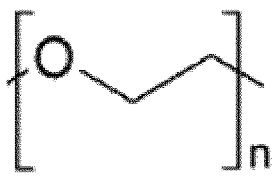

dans laquelle n est compris dans la plage allant de 10 à 2 400.

**13.** Procédé selon la revendication 11, dans lequel le conjugué séparé à l'étape (b) a une structure dans laquelle le lieur non peptidylique est lié à l'extrémité N-terminale du fragment Fc d'immunoglobuline.

**14.** Procédé selon la revendication 11, dans lequel l'étape (b) comprend la détermination d'un rapport de liaison du conjugué et d'un rapport de liaison du fragment Fc d'immunoglobuline à l'aide de l'équation avant séparation du conjugué polypeptide physiologiquement actif-fragment Fc d'immunoglobuline.

**15.** Procédé selon la revendication 11, dans lequel le polypeptide physiologiquement actif est choisi dans le groupe constitué par l'insuline, les interférons, l'hormone de croissance humaine (hGH) et un agoniste des récepteurs de GLP-1.

**16.** Procédé selon la revendication 11, dans lequel

(a) le fragment Fc d'immunoglobuline comprenant la région se liant à FcRn comprend un domaine CH2, un domaine CH3, ou les deux ;
(b) le fragment Fc d'immunoglobuline est sous une forme non glycosylée ;
(c) le fragment Fc d'immunoglobuline comprend en outre une région charnière ;
(d) le fragment Fc d'immunoglobuline est un fragment Fc dérivé du groupe constitué par l'IgG, l'IgA, l'IgD, l'IgE, l'IgM, des combinaisons de celles-ci, et des hybrides de celles-ci ; ou
(e) le fragment Fc d'immunoglobuline est un fragment Fc de l'IgG4.

FIG 1.

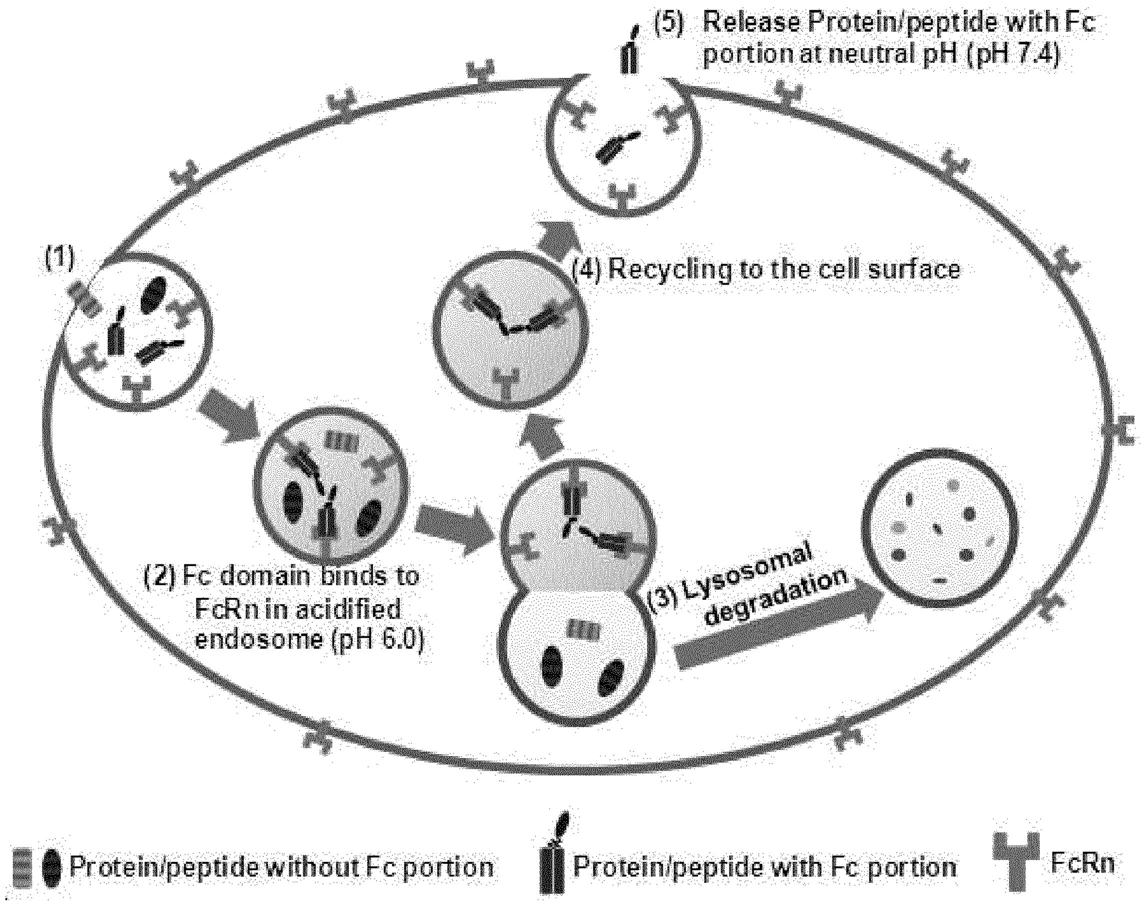
(5) Release Protein/peptide with Fc portion at neutral pH (pH 7.4)
(1)
(4) Recycling to the cell surface
(2) Fc domain binds to FcRn in acidified endosome (pH 6.0)
(3) Lysosomal degradation
Protein/peptide without Fc portion
Protein/peptide with Fc portion
FcRn

FIG 2.

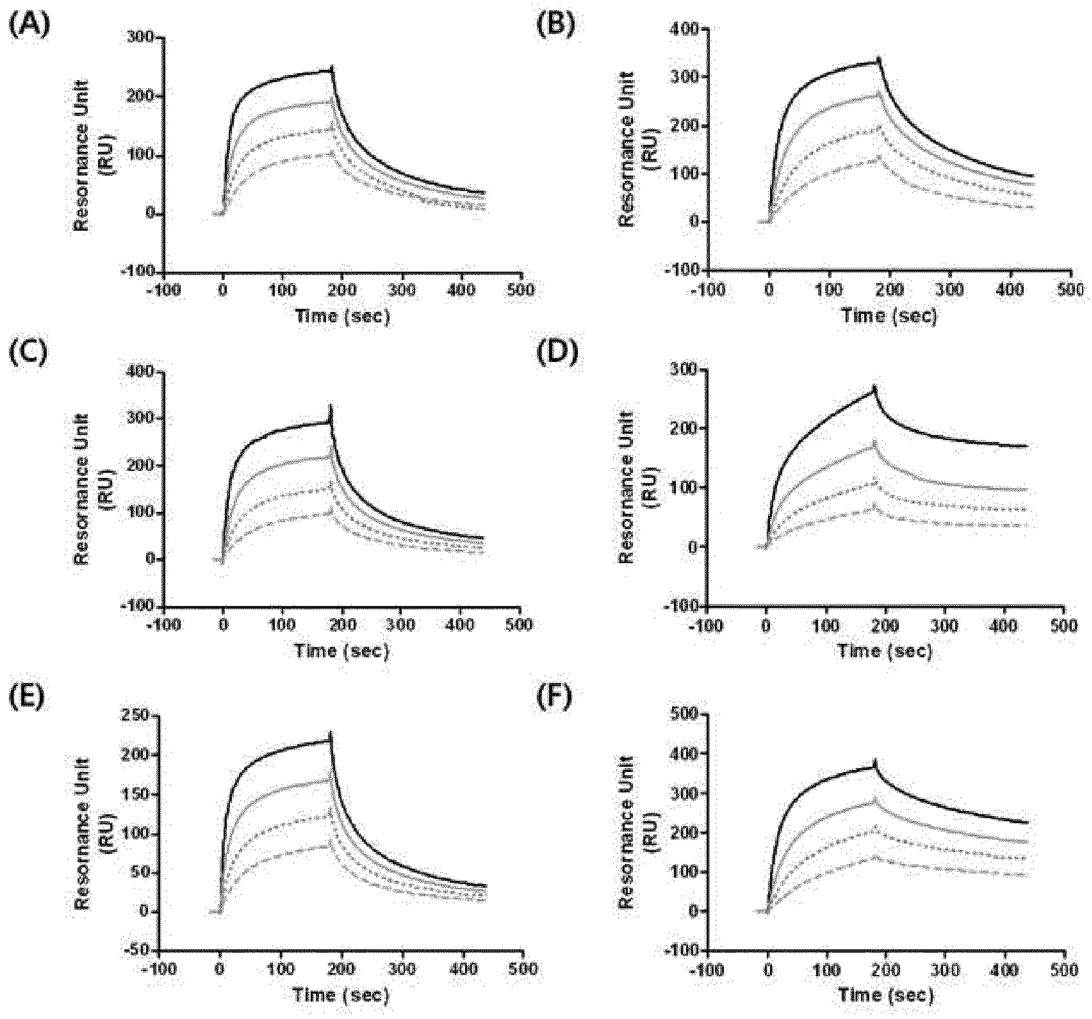

FIG.3

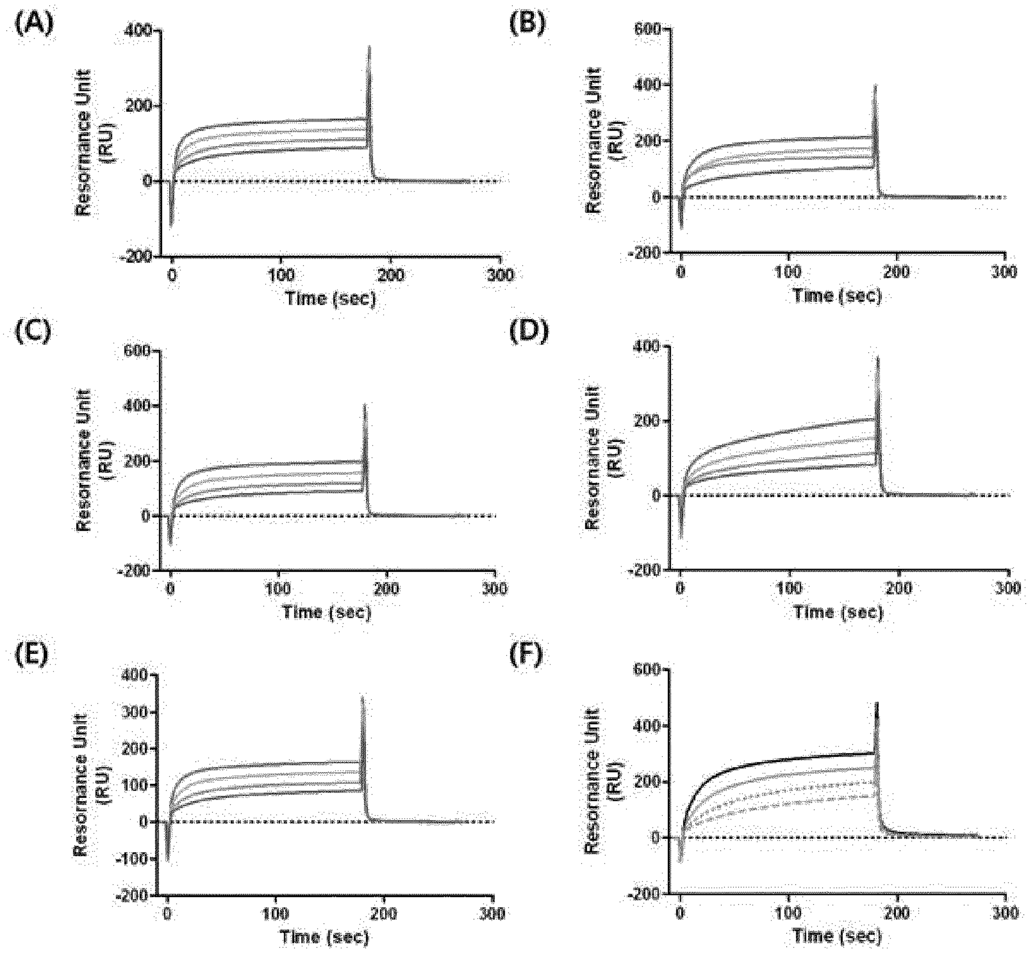

FIG.4

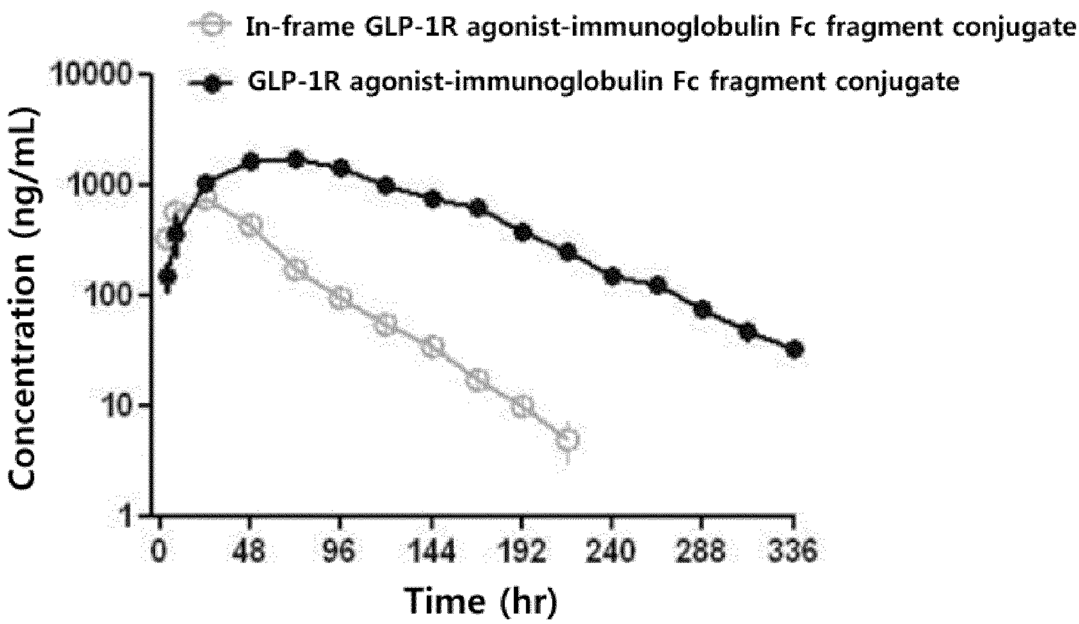
In-frame GLP-1R agonist-immunoglobulin Fc fragment conjugate
GLP-1R agonist-immunoglobulin Fc fragment conjugate
Concentration (ng/mL)
10000
1000
100
10
1
0
48
96
144
192
240
288
336
Time (hr)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020120137271
- KR 1020090008151
- WO 9734631 A
- WO 9632478 A
- KR 1020060077377


### Non-patent literature cited in the description


- **WEIRONG WANG et al.** *DRUG METABOLISM AND DISPOSITION,* 2011, vol. 39, 1469-1477
- **H. NEURATH ; R. L. HILL.** The Proteins, Academic Press. 1979